# NEW EUROPEAN PATENT SPECIFICATION

(11) **EP 0 321 488 B2**
(45) Date of publication and mention of the opposition decision: **26.06.1996**
(45) Mention of the grant of the patent: 14.07.1993
(21) Application number: 87905772.7
(22) Date of filing: 08.09.1987
(51) Int. Cl.: C12N 15/60, C12N 9/88

(54) **PRODUCTION OF PHENYLALANINE AMMONIA LYASE**
HERSTELLUNG VON PHENYLALANIN-AMMONIALYASE
PRODUCTION D'AMMONIALYASE DE PHENYLALANINE (PAL)

(30) Priority: 08.09.1986 GB 8621626
(43) Date of publication of application: 28.06.1989
(73) Proprietor: Microbiological Research Authority, Salisbury, Wiltshire SP4 0JG (GB)
(72) Inventor: ANSON, John, Wiltshire (GB); GILBERT, Harold, Gateshead,Tyne and Wear (GB); ORAM, Jon, Bohune,Pewsey,Wiltshire (GB); MINTON, Nigel, Peter, Wiltshire (GB)
(74) Representative: Skelton, Stephen Richard
(86) International application number: GB8700628
(87) International publication number: WO8802024

(56) References cited:
- EP-A- 0 137 280
- Gene, vol. 36, Elsevier Science Publishers 1985, M. Tully et al "Transformation of rhodosporidium toruloides", pages 235-240
- Journal of Bacteriology, vol. 161, no.1, January 1985, American Society for Microbiology, H.J. Gilbert et al "Molecular cloning of the phenylalanine ammonia lyase gene from rhodosporidium toruloides in Escherichia coli K-12", pages 314-320, 2-21
- Drug Development Research, vol. 1, 1981, Alan R. Liss Inc., W.L. Miller et al "Synthesis of biologically active proteins by recombinant DNA technology", pages 435-454
- Nucleic Acids Research, vol. 10, no.12, 1982, IRL Press Ltd (Oxford, GB) R.Derynck et al "Human interferon is encoded by a single class of mRNA", pages 3605-3615
- Chemical Abstracts, vol.104, 1986 (Columbus, Ohio, US) H.J. Gilbert et al "Cloning and expression of the Erwinia chrysanthemi asparaginase gene in Escherichia coli and Erwinia carotovora", see page 178, abstract no.103508s & J. Gen. Microbiol. 1986, 132 (1), 151-60
- Chemical Abstracts, vol. 97, no.1, 5 July 1982 (Columbus, Ohio, US) H.J. Gilbert et al "Synthesis and degradation of pheniylalanine ammoniolyase of Rhodosporidium toruloides", see page 338, abstract no.3335b & J. Bacteriol. 1982, 150(2), 498-505
- Chemical Abstracts, vol. 98, no.17, 25 April 1983 (Columbus, Ohio, US) H.J. Gilbert et al "Control of synthesis, of functional mRNA coding for phenylalanine ammonio-lyase from rhodosporidium toruloides", see page 315, abstract no.140317g & J. Bacteriol. 1983, 153(3), 1147-54
- Rasmussen, O.F. et al. DNA Sequence (1991)1,3 p. 207-213

## Description

This invention relates to genetic material which encodes the protein phenylalanine ammonia lyase (herein abbreviated to 'PAL') and in particular to such genetic material which lacks the intervening non-coding DNA (introns) normally found in the PAL - encoding gene in its natural state.

Phenylalanine ammonialyase (PAL; EC 4.3.1.5) which occurs in plants, yeasts, fungi, and streptomycetes catalyzes the nonoxidative deamination of L-phenylalanine to trans-cinnamic acid (see Gilbert et al., 1985). The enzyme has a potential role in the treatment and diagnosis of phenylketonuria (Ambrus et al., 1978) and has industrial applications in the synthesis of L-phenylalanine from trans-cinnamic acid (Yamada et al., 1981). In plants the enzyme, involved in flavanoid biosynthesis, is induced by illumination while in gherkin and mustard seedlings induction is the result of activation of a constitutive pool of inactive enzyme (Attridge et al., 1974). Illumination elicits de novo synthesis of the enzyme in other botanical species (Schroder et al., 1979). Gherkin, apple, sweet potatoe, and sunflower PAL is also regulated by a specific inactivating system (Tan, 1980).

In some basidiomycete yeast phenylalanine can act as sole source of carbon, nitrogen, and energy. As PAL catalyzes the initial reaction in the catabolism of the amino acid, the enzyme plays a key role in regulating phenylalanine metabolism. In Rhodosporidium toruloides PAL is induced by the presence of L-phenylalanine or L-tyrosine (Marusich et al.. 1981). Glucose, and ammonia in the presence of glucose, repress PAL synthesis (Marusich et al., 1981), while induction of PAL activity is the result of de novo synthesis of the enzyme rather than activation of an inactive precursor or a decrease in the rate of PAL degradation (Gilbert and Tully, 1982). Glucose represses PAL synthesis but has no effect upon stability of the enzyme, whereas ammonia prevents uptake of phenylalanine and so may repress enzyme synthesis through inducer exclusion (Gilbert and Tully, 1982). In vitro translation data of mRNA, isolated from R. toruloides grown under different physiological conditions, showed that phenylalanine, ammonia and glucose regulate PAL synthesis by adjusting the level of functional PAL mRNA (Gilbert et al., 1983).

In recent years genetic engineering methods have been developed whereby microorganisms which are common or which can easily be grown on an industrial scale, in particular certain bacteria or yeasts. have their genetic material (DNA sequences) modified so that they produce a desired compound eg a protein. Broadly this is achieved by inserting into the host microorganism a plasmid consisting of a gene which is a polynucleotide sequence which encodes the compound. together with other genetic material which instructs the host's genetic apparatus to synthesise the compound.

The gene encoding PAL has recently been cloned as a 8.5 kb genomic Pstl fragment (Gilbert et al., 1985). These studies indicated that PAL is synthesised from a monocistronic mRNA of 2.5 kb, and that the gene is present as a single copy in the the R. toruloides genome. The introduction of the cloned PAL gene into both E. coli (Gilbert et al., 1985) and Saccharomyces cerevisae (Tully and Gilbert, 1985) did not result in the production of PAL protein.

Although attempts have been made along these lines to introduce the cloned PAL - encoding gene from R-toruloides into the microorganism E-coli (Gilbert et al; 1985) and into the yeast Saccharomyces Cerevisae (Tully and Gilbert, 1985), these heterologous hosts did not then produce PAL protein.

It is an object of the invention to provide genetic material which may be introduced into host organisms other than R-toruloides, which hosts will then produce PAL protein. Other objects and advantages of the invention will be apparent from the following description.

According to a first aspect of the invention there is provided an intron-free structural gene, derived from a corresponding intron-containing structural gene from a eukaryotic microorganism, both genes coding for the same gene product provided that the intron-free gene is capable of expressing the product within a prokaryotic or eukaryotic microorganism. The gene product may be a chemical compound the production of which is desired, for example a protein.

According to a second, preferred aspect of the invention there is provided an intron-free structural gene which encodes PAL or a polypeptide which displays PAL activity. The gene is preferably derived from a PAL - producing strain of a eukaryotic organism, most preferably a strain of R toruloides.

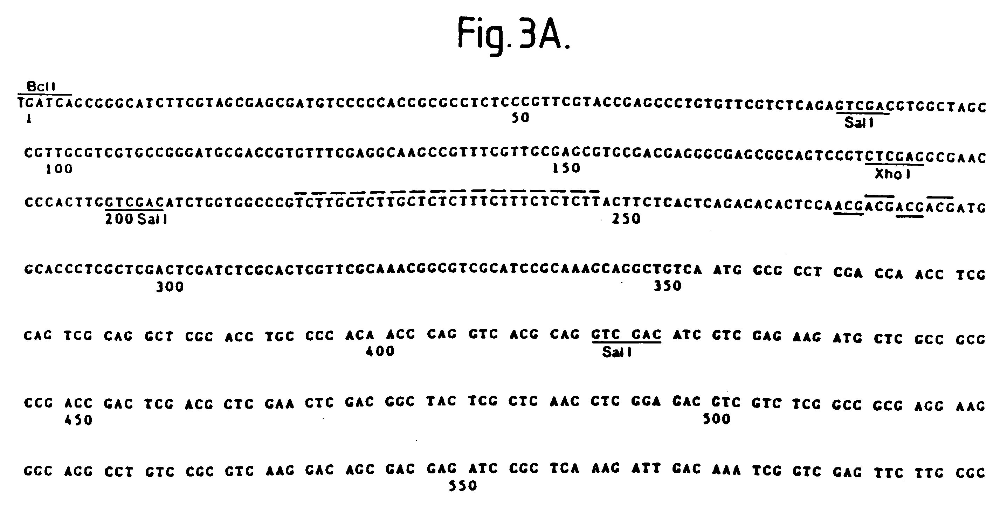

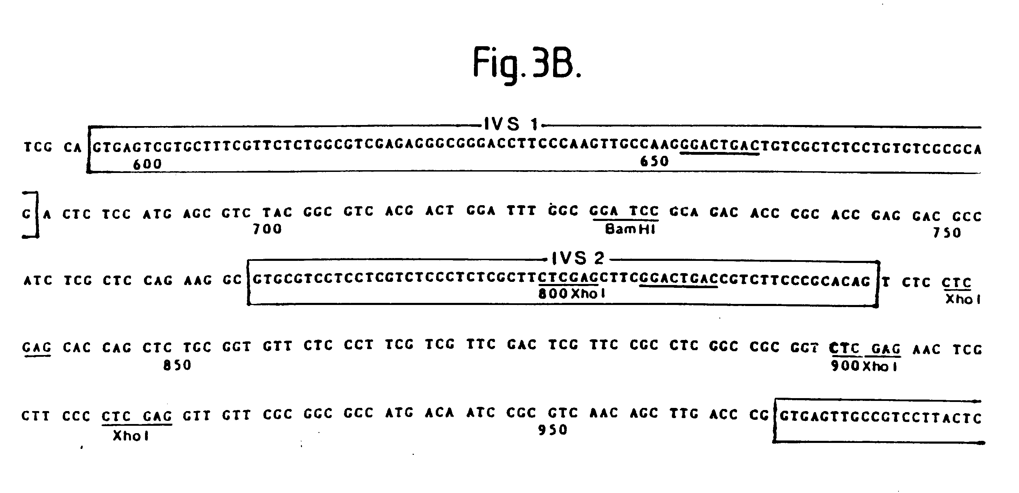

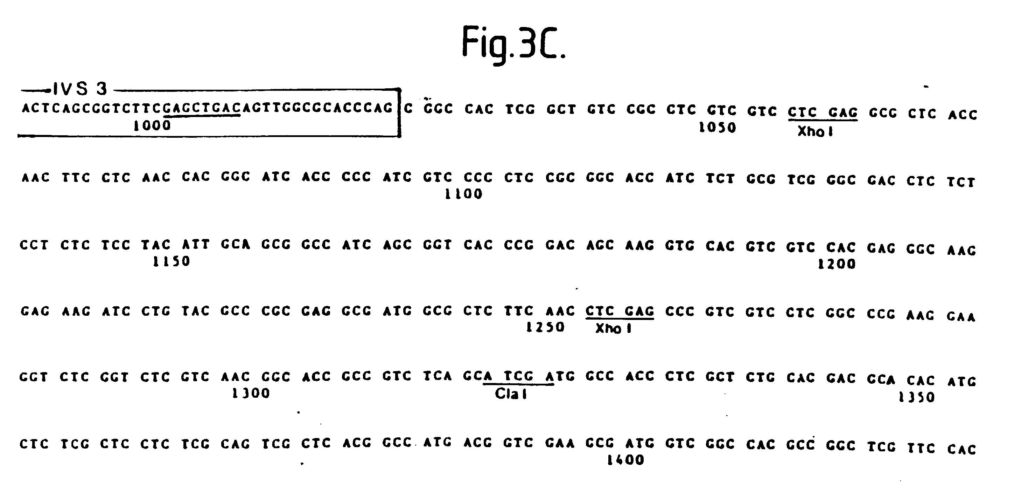

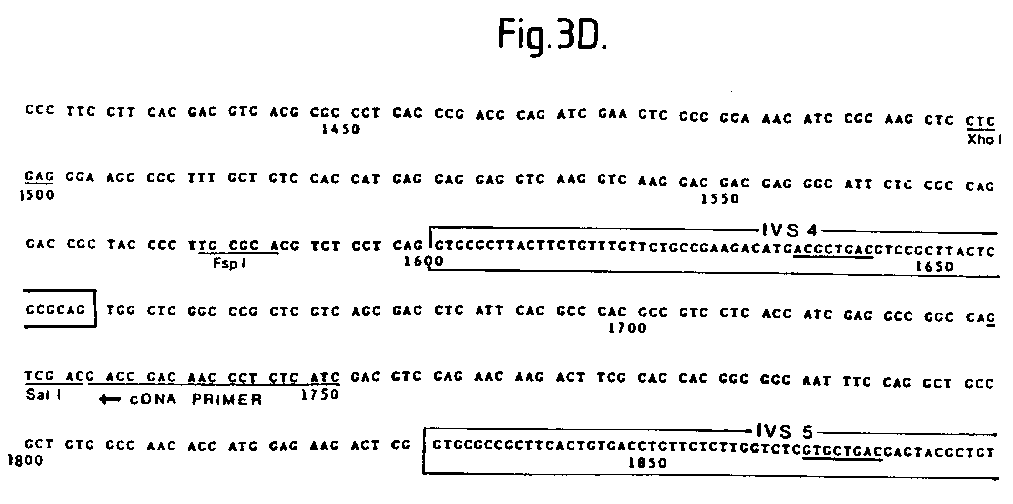

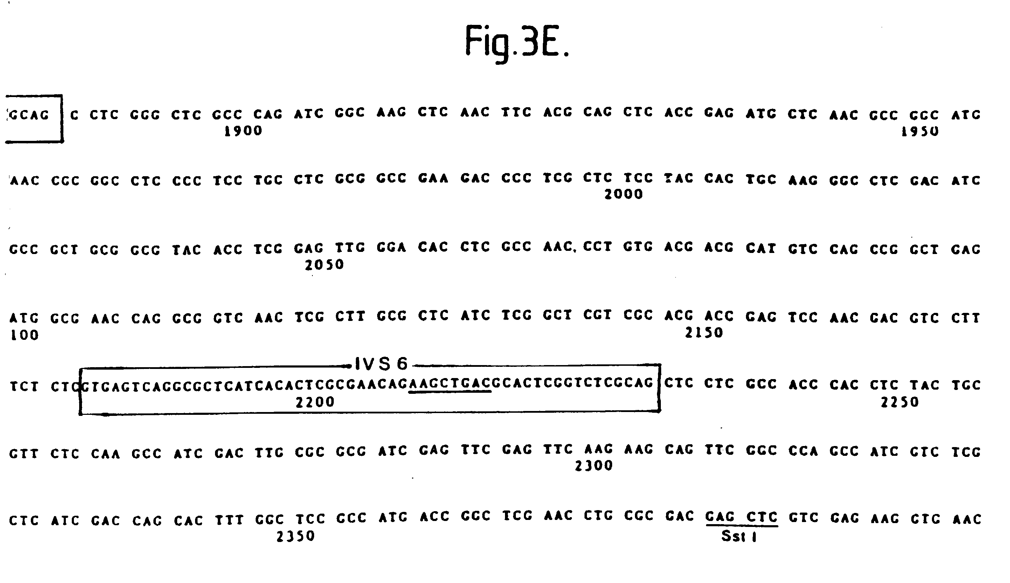

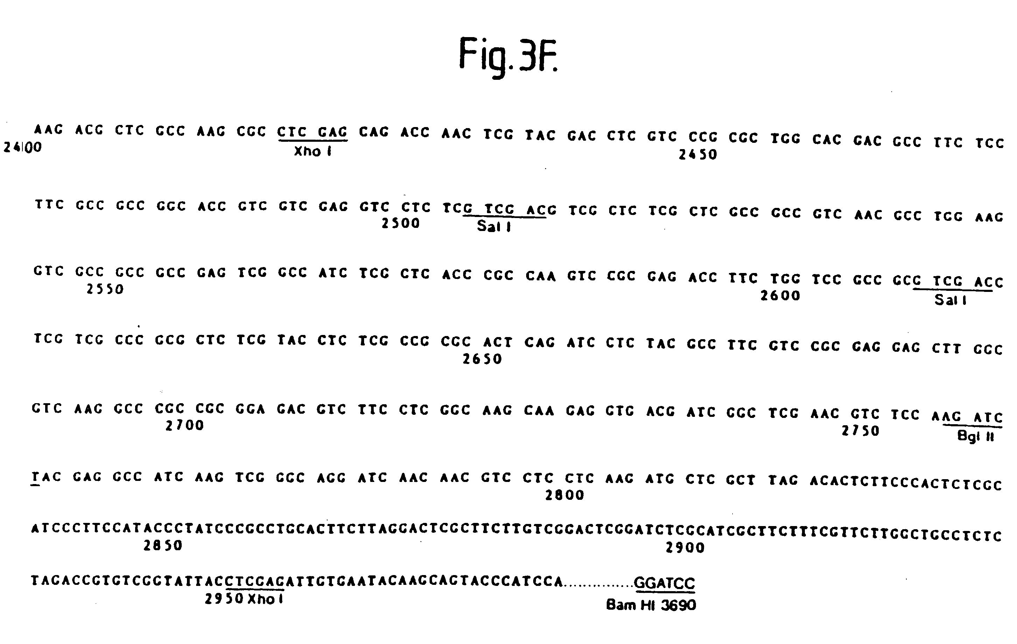

A portion of the genetic DNA polynucleotide sequence of R. toruloides is shown in Fig 3. The methods used by the inventors to determine this sequence are described later. The introns, six in number, are marked IVS 1 to IVS 6. Also shown are the various restriction sites. The gene of the second aspect of the invention therefore preferably consists of an intron free gene characterised in that it has a structure identical to that of the PAL gene present in cDNA obtainable by either: (a) annealing total mRNA from PAL induced R. toruloides cells to oligo dT tailed pUC9, synthesising the first strand cDNA copy using reverse transcriptase in the presence of all 4 dNTPs, tailing the newly synthesised strands with oligo dC using terminal deoxynucleotidyl transferase, fractionating by an alkaline sucrose gradient, annealing single stranded plasmid DNA carrying cDNA sequences to denatured oligo dG tailed pUC9 and synthesising the second strand with Klenow DNA polymerase in the presence of all four dNTPs; or (b) synthesising a cDNA strand from total mRNA using reverse transcriptase and a 19-mer primer of sequence GATGAGAGGGTTGTCGGTC complementary to PAL mRNA, nicking the RNA in the RNA-DNA hybrid so produced with RNAaseH and replacing it by DNA using E.coli DNA polymerase using the nicked RNA as a primer, or a gene having a nucleotide sequence which codes for the same amino acids as this sequence.

It is well known in the field of genetics that DNA sequences which are related to or derived from a defined sequence may encode the same protein or a polypeptide having similar activity to that expressed by the defined sequence. For example the related or derived sequence may lack some bases or may include some additional bases. Also it is known that the genetic code is degenerate, in that several codons may encode the same amino acid. The related or derived sequence may therefore contain some codons which are different to those listed in Fig 3 but which preferably encode the same amino acid. Genes which are related to or derived from this sequence of codons in one or more of these ways are included in the invention.

Genes related to or derived from this sequence may also be defined in terms of the degree of conformity to this sequence. This is preferably as high as possible, ideally 100%, but 70% or higher, eg 85% or higher conformity to that sequence is generally satisfactory.

To enable a gene according to the first or second aspects of the invention to be introduced into a host organism, it is common to include the gene into a recombinant DNA molecule. According to a third aspect of the invention there is therefore provided a recombinant DNA molecule, especially a plasmid, which contains a gene according to the first or second aspects of the invention.

The plasmid according to this aspect of the invention may be used as a vector to introduce the gene into a host and may therefore also contain additional genetic material appropriate to a host into which it is intended to introduce the plasmid. Such genetic material may preferably contain an expression control sequence operatively linked to said gene, and/or transcription/translation signals from other genes appropriate to the organism into which the plasmid is to be introduced and from which expression of the product, eg PAL, is hoped.

The structure of the plasmid according to this aspect of the invention will vary according to the host organism for which it is to be used as a vector, but by positioning the gene of the first or second aspect of the invention downstream of the appropriate regulatory signals, vectors may be prepared using which expression of R. toruloides PAL may be obtained in any of the currently used production organisms. These include E. coli K12, Bacillus subtilis, Saccharomyces cerevis ae, Pseudomonas putida, Erwinia chrysanthemi and mammalian cell lines.

Similarly the nature of the regulating DNA sequences immediately upstream of the PAL cDNA coding region in the plasmid will be composed of appropriate, characterised transcription/translation signals. For example for use in S. cerevisae a ribosome binding site (conforming to the sequence CCACCTT) may be positioned at the appropriate position upstream of the translational start of the PAL gene, and powerful transcriptional signals, such as those derived from the S. cerevisae phosphoglycerate kinase and mating facter genes, placed 5' to the ribosome binding site. The plasmid itself may use standard replicons (eg 2p) and selectable markers (e.g. Leu2, Trp etc). Similarly, for use in E. coli use will be made of the PL, tac trp, rac or lac promoters, with appropriate bacterial ribosome binding sites, and plasmids based on ColEl (e.g. PBR32 and pUC plasmids), RSF1010, and runaway replicons of RI. As the introns present in the natural PAL gene act as a barrier to the expression of PAL in organisms other than R. toruloides, the invention may be used to produce PAL in a wide range of procaryotic and eukaryotic hosts which are unable to express the natural PAL gene due to the presence of the 6 introns.

In accordance with a fourth aspect of the invention there is provided a host organism, especially a strain of E. coli, Erwinia sp., Clostridia sp., Streptomyces sp., B. subtilis, B. stearothermophilus, Pseudomonas, other microorganisms such as bacilli, yeasts, other fungi, animal or plant hosts, and preferably a prokaryotic host, transformed with at least one recombinant DNA molecule according to the third aspect.

The invention also provides a process for the preparation of a gene from which introns have been deleted which includes the steps of:
(i) isolating PAL mRNA from a strain of R. toruloides,
(ii) synthesising two intron-free complementary DNA ('cDNA') sequences from the mRNA, the two cDNA sequences each containing a portion of a gene which encodes PAL or a polypeptide which displays PAL activity, the two portions together containing the 3' and the 5' ends of the gene.
(iii) joining the two cDNA sequences proposed in (ii) to form an intron-free structural gene which encodes PAL or a polypeptide which displans PAL activity.

The method used in step (ii) may use a cloning method which forms the cDNA sequences contained in plasmids. In such a case the sequences may be isolated from the plasmids which contain them by cleavage of the plasmids at a suitable restriction site, followed by ligation of the two sequences to form the gene. The gene may then be combined with other genetic material to form a plasmid containing it for example following cleavage of a suitable known plasmid such as pUC9 at appropriate sites. If desired the gene may then in turn be excised from this plasmid and combined with yet other genetic material to form other plasmids which may be used as vectors. Standard recombinant DNA techniques, familiar to those skilled in the art may be used for the process of the invention.

The gene and/or plasmid produced in step (iii) of this process is preferably one of the genes or plasmids encompassed by the second and/or third aspects of the invention, and the cDNA sequences produced in step (ii) are consequently preferably portions of these. The cDNA sequences produced in step (ii) and plasmids containing them are further aspects of the invention.

The invention therefore also includes DNA polynucleotide sequences, eg plasmids, the same as or substantially the same as or derived from or related to those produced by the process of the invention.

The invention will now be described by way of examole only with reference to the accompanying figures:
- Fig 1: is a schematic diagram illustrating how the genetic DNA carrying the PAL gene was sequenced.
- Fig 2: illustrates the production of the two plasmids carrying the PAL gene which lack the intron sequences of the natural gene.
- Fig 3: shows the complete nucleotide sequence of the genomic clone, the intron sequences removed in the invention being labelled IVS1 to IVS6,
- Fig 4: shows the formation of the recombinant plasmid pPAL 3 containing the intron-free gene, by combination of the two cDNA plasmids pPAL1 and pPAL2.
- Figs 5 & 6: illustrates the DNA nucleotide sequences of the overlapping cDNA clones pPAL1 and pPAL 2 respectively.
- Fig 7: shows the expression of PAL protein from the plasmid pPAL4.
In this description and the figures the following abbreviations are used:

| Amino acid | symbol | Nucleotide bases | symbol |
|---|---|---|---|
| Alanine | Ala | Uracil | U |
| Arginine | Arg | Thymine | T |
| Asparagine | Asn | Cytosine | C |
| Aspartic acid | Asp | Adenine | A |
| Asn + Asp | Asx | Guanine | G |
| Cysteine | Cys | | |
| Glutamine | Gln | | |
| Glutamic Acid | Glu | | |
| Gln + Glu | Glx | | |
| Glycine | Gly | | |
| Histidine | His | | |
| Isoleucine | Ile | | |
| Leucine | Leu | | |
| Lysine | Lys | | |
| Methionine | Met | | |
| Phenylalanine | Phe | | |
| Proline | Pro | | |
| Serine | Ser | | |
| Threonine | Thr | | |
| Tryptophan | Trp | | |
| Tyrosine | Tyr | | |
| Valine | Val | | |

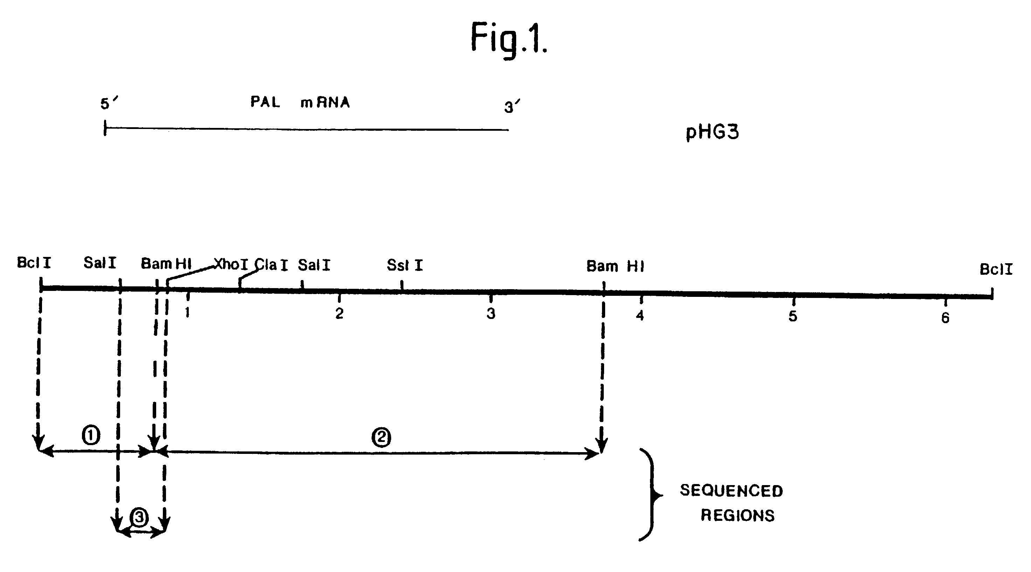

### Referring to Figs 1 to 6 in more detail:

In Fig 1. Region 2 was isolated from the appropriate clone (pHG3), circularised by treatment with T4 DNA ligase, fragmented by sonication, and fragments of between 500 and 1000 bp inserted into M13mp8. Region (1) was inserted into M13mp8 and M13mp9 as various specific fragments utilising the restriction sites BamHI, Bcll and Sall. The sequence of the DNA spanning the BamHI site was obtained by coning the indicated fragment (3) into M13mp8.

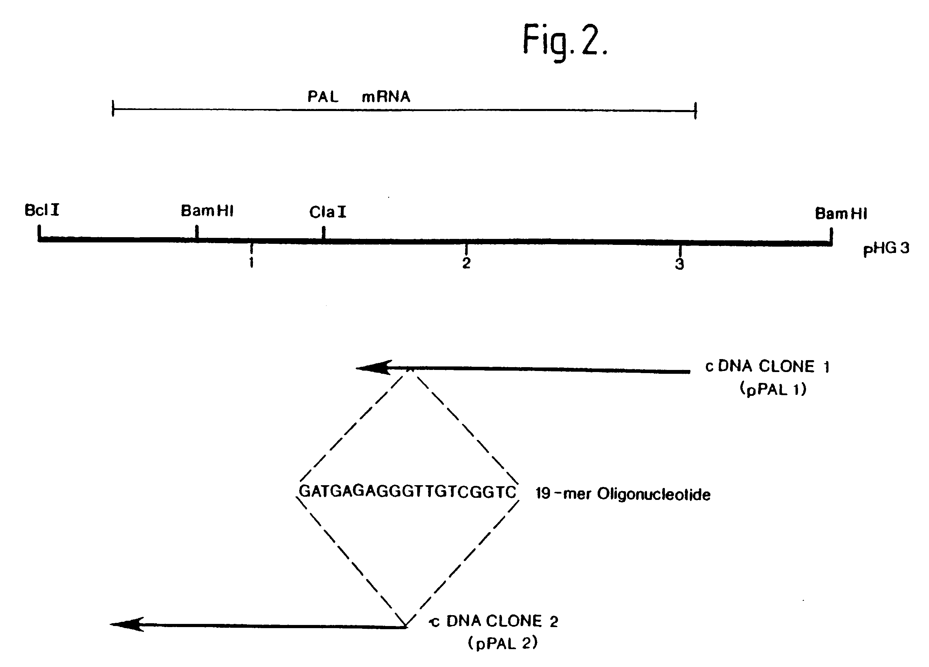

In Fig 2. Clone 1 (pPAL1) was obtained by the method of Heidecker and Messing (1983). Total mRNA from pal-induced R. toruloides cells was annealed to oligo(dT)-tailed pUC9, and the first strand cDNA copy synthesised using reverse transcriptase in the presence of all four dNTP's. The newly synthesised strands were tailed with oligo(dC) using terminal deoxynucleotidyl tranferase. Following fractionation by an alkaline sucrose gradient, single-stranded plasmid DNA carrying cDNA sequences were annealed to denatured oligo(dG)-tailed pUC9 and the second strand synthesised using DNA polymerase (Klenow) and the addition of all four dNTP's. Clone 2 (pPAL2) was constructed using the procedure of Gubler and Hoffman (1982). The first strand cDNA copy was synthesised using reverse transcriptase and a 19-mer oligodeoxynucleotide primer (GATCAGAGGGTTGTCGGTC) complementary to pal mRNA. The RNa within the RNA-DNa hybrid was then nicked with RNase H and the RNA strand replaced with DNA by E. coli DNA polymerase, utilsing the nicked RNA as a primer. The double stranded DNA was then blunt ended by the action of T4 DNA polymerase, tailed with oligo(dC), and annealed to oligo(dG) tailed pBR322. cDNA clones produced using both methods were transformed into E. coli JM83, and colonies screened for Pal cDNa sequences using [α-³²p] dATP-labelled pHG3 restriction fragments.

In Fig 3. The determined amino acid sequences of the 5 randomly derived peptide fragments are indicated by overlining of the relevant residues. The introns are labelled IVS 1-6, and the sequence common to all 6 is indicated by underlining of the relevant region. A dashed overline in the 5' non-coding region represents the TC rich region of the sequence, while the under- and overlining immediately downstream marks a repetitive region. The sequence extends from the most leftward Bcll site of Figure 1, to the 3' end of cDNA clone PPAL1 (Figure 2).

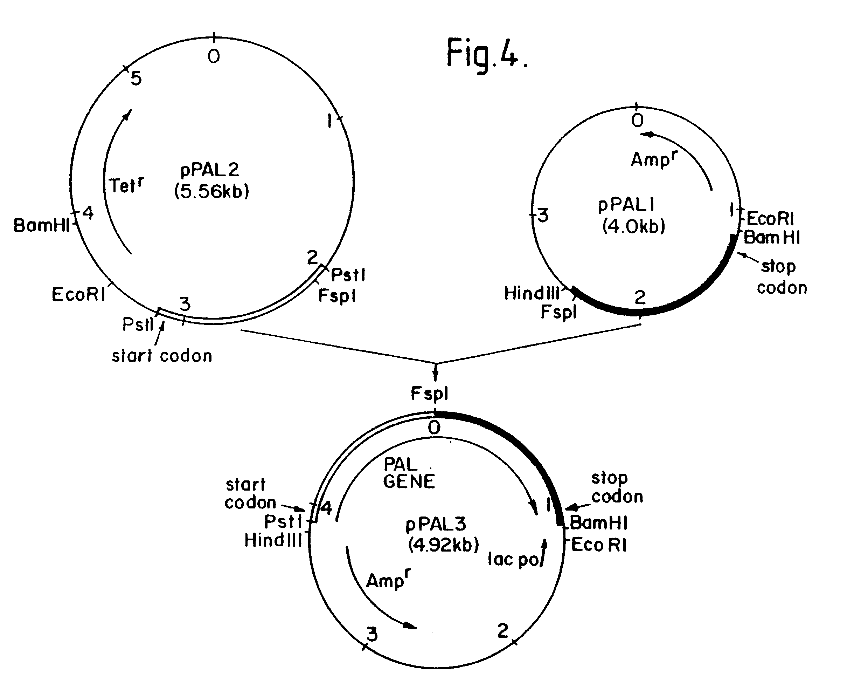

In Fig 4. The single line of the pPAL1 and pPAL3 circular maps represent pUC9 and pUC8 derived DNA, respectively, while the single line of pPAL2 represents pBR322 DNA (see Fig. 2 for construction of pPAL1 and pPAL2). The double line of pPAL2 represents the "intron-free" 5' end of the PAL gene, while the thick line of pPAL1 represents the 3' end of the gene. The 5' end of the PAL gene was isolated as a 1.0kb Pst1 - Fsp1 fragment from pPAL2 and ligated to a 1.25kb Fsp1 - BamHI fragment, isolated from pPAL1, which carried the 3'end of the gene. The ligated fragment was inserted between the BamHl and Pstl sites of pUC8 to yield pPAL3. The positions of the PAL gene translational start (ATG) and stop (TAG) codon (see Fig. 3) are marked by arrows. The orientation of insertion of the PAL gene is such that transcriptional read through from the vector borne lac promoter (lac po) will not occur.

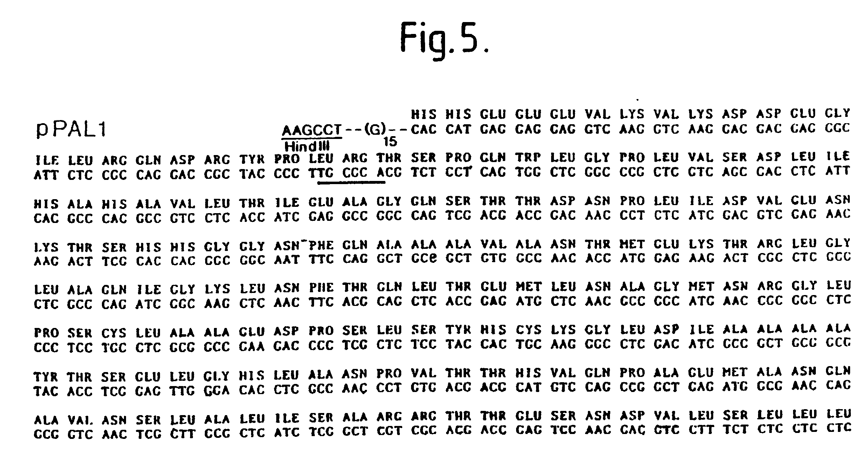

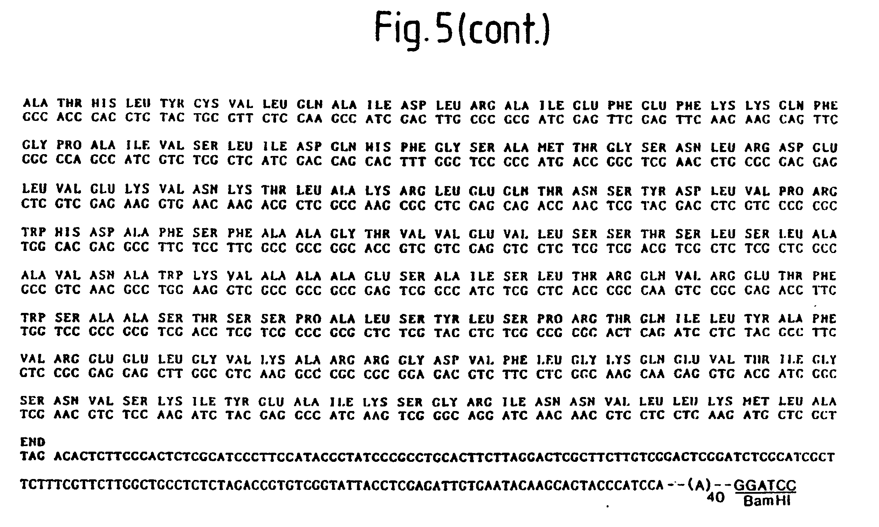

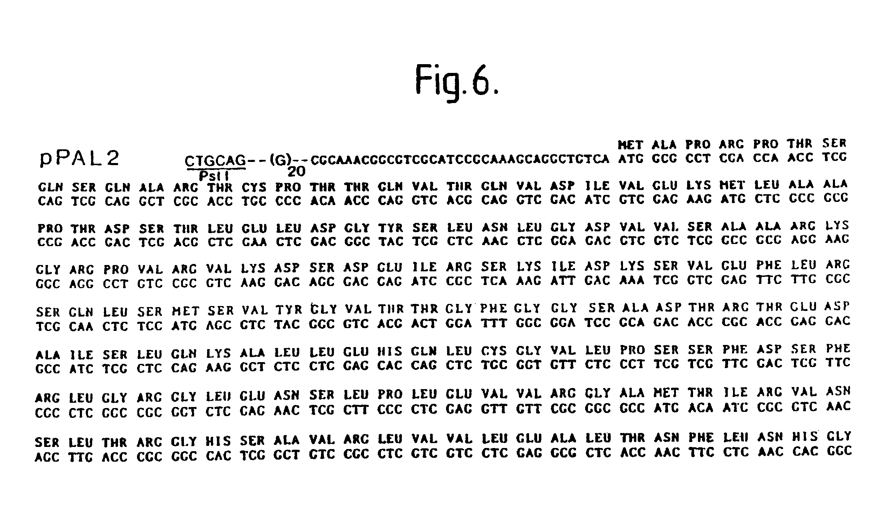

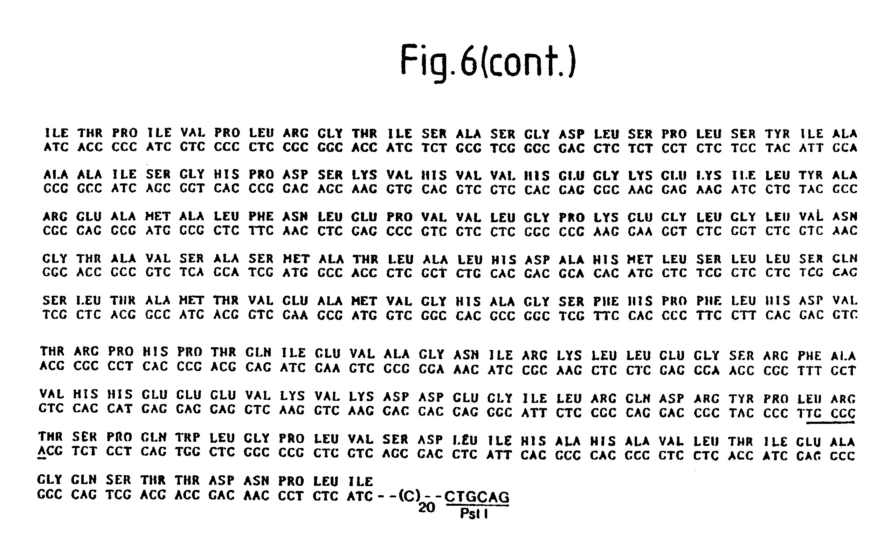

In Fig 5 & 6. The Fsp 1 site-used to join these two clones to form pPAL 3 is indicated by underlining of the relevant nucleotides.

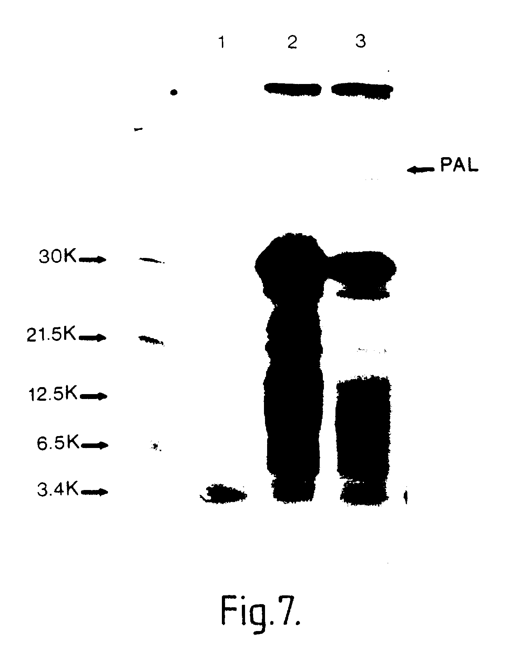

In Fig 7. The plasmid pPAL4 contains the complete PAL gene from pPAL 3 (Fig 4) cloned into pUC9 as an EcoRI - Hind III fragment such that transcriptional read through from the adjacent lac promoter can occur. Gene product formation was assessed using a plasmid-directed in vitro translation kit obtained from Amersham International PLC. Samples in the numbered tracks are as follows; 1, no DNA added; 2, plasmid pUC9; 3, plasmid pPAL4. Molecular weights of the protein markers are given as M,.

In the following description reference will be made to the following general procedures:

### MICROBIAL STRAINS AND PLASMIDS

Microbial strains and plasmids used in accordance with the invention are listed in Table 1.

### MEDIA

E. coli strains were cultured in L-broth (1% tryptone, 0.5% yeast extract, 0.5% NaCI). Media were solidified with the addition of 2% (w/v) Bacto-agar (Difco). Ampicillin (100 µg ml⁻¹ was used for the selection and growth of transformants. Functional β -galactosidase was detected by the addition of 5-bromo-4-chloro-indoyl- β -D-galactoside (X-Gal) to a final concentration of 2 µg ml⁻¹

### CHEMICALS

[α ³²P] dATP and the cDNA synthesis kit were obtained from Amersham International. Agarose, restriction enzymes, T4 DNA ligase, terminal deoxynucleotidyl transferase and 17mer universal sequence primer were purchased from Bethesda Research Laboratories. Klenow DNA polymerase was from Boehringer Mannheim, while dT tailed pUC9 was from PL-Biochemicals. Reverse transcriptase was purchased from Anglicon Biotechnology Ltd. while all other reagents were obtained from Sigma Chemical Co. or BDH.

**TABLE 1**

| **Microbial Strains and Vectors Used** | | |
|---|---|---|
| Strain | | |
| E. coli JM83 | ara, (lac-pro) rpsL, thi, 080d lacI ZM15 | Vieria and Messing (1982) |
| E. coli JM101 | (lac-pro), supE, thi/ FlacI ZM15 traD pro | Messing and Vieria (1982) |
| Plasmids | | |
| pUC9 | Amp^{R} | Vieria and Messing (1982) |
| pBR322 | Amp^{R} Tet^{R} | Bolivar et al. (1977) |
| pGH3 | Amp^{R} (PAL genomic clone) | Gilbert et al. (1985) |
| pPAL1 | Amp^{R} (3' end PAL cDNA clone) | Novel plasmids |
| pPAL2 | Amp^{R} (5' end PAL cDNA clone) | Novel plasmids |
| pPAL3 | Amp^{R} (entire PAL cDNA gene) | Novel plasmids |
| pPAL4 | Amp^{R} (entire PAL cDNA gene) | Novel plasmids |
| Bacteriophage | | |
| M13mp8 | | Messing and Vieria (1982) |
| M13mp9 | | Messing and Vieria (1982) |

### DNA MANIPULATIONS

All restriction enzymes and DNA/RNA modifying enzymes were used in the buffers and under the conditions recommended by the suppliers. Plasmid transformation techniques and all manipulation of DNA have previously been described (Minton et al., 1984).

### PLASMID DNA ISOLATION

E. coli plasmids were purified from 1 litre of L-broth cultures containing ampicillin by "Brij lysis" and subsequent CsCI density gradient centrifugation (Clewell and Helinski, 1969). The rapid boiling method of Holmes and Quigley (1981) was employed for small scale plasmid isolation screening purposes.

### TEMPLATE GENERATION BY SONICATION

The DNA to be sequenced was fragmented into random blunt-ended fragments by the procedure of Deininger (1983). The fragments obtained were cloned into the Smal site of M13mp8 and template DNA prepared as described by Sanger et al (1980).

### NUCLEOTIDE SEQUENCING

Nucleotide sequencing was undertaking by the dideoxy method of Sanger et al (1980). The data obtained was compiled into a complete sequence using the computer programmes of Staden ( 1980 ).

### ISOLATION OF PAL mRNA

PAL mRNA was isolated as has previously been described (Gilbert et al., 1985) employing publicly available R. toruloides strain IFO 0559 (equivalent to NCYC 1589 deposited at the National Collection of Yeast Cultures, Norwich (GB) under the terms of the Budapest Treaty on 8 September 1986).

### CDNA CLONING (SYNTHESIS)

### i) Heidecker-Messing Method

The method utilised was essentially as described by Heidecker and Messing (1983). Total mRNA from PAL induced R. toruloides cells was annealed to oligo dT tailed pUC9 and the first strand cDNA copy synthesised using reverse transcriptase in the presence of all 4 dNTP's. The newly synthesised strands were tailed with oligo dC using terminal deoxynucleotidyl transferase. Following fractionation by an alkaline sucrose gradient, single stranded plasmid DNA carrying cDNA sequences were annealed to denatured oligo dG tailed pUC9 and the second strand synthesised using Klenow DNA polymerase and the addition of all 4 dNTP's.

### ii) Gubler-Hoffman Method

The second method employed in the synthesis of cDNA was that of Gubler and Hoffman (1983). The first strand cDNA copy was synthesised using reverse transcriptase and a 19-mer primer (GATGAGAGGGTTGTCGGTC) complementary to PAL mRNA. The RNA within the RNA-DNA hybrid was then "nicked" with RNaseH and the RNA strand replaced with DNA by E. coli DNA polymerase, utilising the nicked RNA as a primer. The double stranded DNA was then blunt-ended by the action of T4 DNA polymerase, tailed with oligo dC and annealed to oligo-dG tailed pBR322.

### DETECTION OF PAL cDNA CLONES

Plasmid DNA carrying cDNA inserts were transformed into E. coli JM83 and the Amp^{R} transformants screened for PAL specific DNA. This was undertaken by in situ colony hyridisation (Grunstein and Hogness, 1975), utilising radio labelled pHG3 DNA subfragments carrying portsion of the PAL gene.

### AMINO ACID SEQUENCING

Peptide fragments of purified (according to Gilbert et al., (1985) PAL protein were isolated as previously described (Minton et al., 1984). Amino acid sequencing was undertaken using automated Edman degradation using an Applied Biosystems gas phase sequencer, model 470A.

### IN VITRO TRANSLATION

The bacterial ion-free coupled transcript ion-translation system used was a modification of that first described by De Vries and Zubay (1967). The E. coli S-30 extract and the supplement solutions required for in vitro expression of genes contained on a bacterial plasmid were purchased as a kit from Amersham International PLC. Proteins produced were labelled with ³⁵S-methionine (Amersham), and analysed by SDS-PAGE on 12% acrylamide gels (Laemmli, 1970). Gels were dried prior to autoradiography for 16 hours.

### 1. NUCLEOTIDE SEQUENCING OF THE PAL GENOMIC CLONE

The PAL gene was previously shown (Gilbert et al., 1985) to occupy a 2.5 kb region of DNA within a 6.7 kb BcII fragment cloned into pUC8 to yield the recombinant plasmid pHG3 (see Fig. 1). The majority of the gene resided on a 3 kb BamHI fragment, while the remaining 5' end of the gene lay on a 0.7 kb BamHI-Bcll fragment. Accordingly, the 3 kb fragment was isolate from an appropriate clone (fragment 2, Fig. 1) and random subfragments, generated by sonication (Deininger, 1983), cloned into M13mp8. A total of some 250 templates were prepared and sequenced, the data obtained being compiled into a complete sequence using the computer programmes of Staden (1980). The sequence of the 5' end of the gene was obtained by the site directed cloning of the relevant BcII-BamHI, Bcll-Sall and BamHI-SaII fragments (region 1, Fig. 1.) into the appropriate sites of M13mp8 and M13mp9. Sequence determination of the DNA spanning the BamHI site was achieved by cloning the Sall-Xhol fragment (3) indicated in Fig. 1.

The translation of the appropriate DNA strand of the sequenced region indicated that an open reading frame (ORF) capable of coding for PAL was not present. Confirmation that this region does encode PAL, however, was obtained by comparing the translated amino acid sequences with the determined sequence of 5 randomly derived peptide fragments. All 5 peptide sequences were located within the translated sequence but occurred in various translational reading frames (Fig. 2.). The absence of a contiguous ORF suggested that, in common with other fungal genes, the PAL gene contains introns.

### 2. ISOLATION OF cDNA CLONES CARRYING THE PAL GENE

To enable the identification of the PAL intervening sequences we elected to reclone the gene from cDNA. In the initial experiments the procedure of Heidecker-Messing (1983) was adopted, utilising the vector pUC9 and purified PAL mRNA. Clones carrying PAL DNA sequences were identified utilising the 3 kb BamHI-BcII fragment of pHG3 as a DNA probe. The largest clone obtained, pPAL1, contained some 1.3 kb from the 3' end of the PAL gene (Fig. 3). The 5' end of the gene was obtained by cloning C-tailed cDNA, prepared by the method Gubler and Hoffman (1983), into G-tailed pBR322, to yield pPAL2. In this case the primer utilised during first strand synthesis was a synthesised 19-mer oligonucleotide complementary to the PAL coding strand 150 bp downstream from the 5' end of the previously obtained cDNA (see Fig. 3.). The nucleotide sequence of the two cDNA clones was determined by site directed cloning of appropriate restriction fragments into M13mp8 and M13mp9.

### 3. INDENTIFICATION OF THE PAL INTRONS

Sequence determination of the 2 clones confirmed the presence of 6 introns within the PAL coding sequence. Thus the 6 regions of DNA label led IVS1 to IVS6 were completely absent from the appropriate regions of pPAL1 and pPAL2. Examination of the 6 missing regions revealed that they all contained the nucleotides CAG at their 3' ends, exhibiting perfect agreement to the consensus intron accepter sequence generally observed in eukaryotic genes (Mount, 1982). A number of the sequences at the 5' end of some of these introns demonstrated less conformity to the eukaryotic consensus donor sequence (GTA/GAGT). Thus the donor sequences of IVS 2, 4 and 5 were GTGCGT, GTGCGC and GTGCGC respectively. The introns of eukaryotic genes have been generally shown to contain sequences necessary for the accurate splicing of the intervening non-coding regions. Seqeunces conforming to consensus sequences observed in the introns of other eukaryotics (e.g. TACTTAACA in S. cerevisae; see Orbach et al., 1986) are not present in the R. toruloides introns. In their place a sequence is present conforming to the consensus G/ANG/CTGAC (the relevant sequence within each intron has been underlined in Fig. 3). Such a sequence may be specific to R. toruloides and closely related organisms.

The PAL gene has been shown not to express in either E. coli (Gilbert et al., 1985) or S. cerevisae - (Tully and Gilbert, 1985). The reason for lack of expression in the former is undoubtedly due to the presence of introns in the PAL gene. Furthermore, although S. cerevisae is capable of splicing introns, the differences in the nucleotide sequences of the PAL introns and those found in S. cerevisae intron probably explains the inability of this yeast to express the R. toruloides PAL gene.

### 4. DERIVATION OF A CONTIGUOUS cDNA PAL GENE

The procedure utilised in the cloning of the PAL gene from cDNA had resulted in two clones, pPAL1, which carried the 3' end of the gene, and pPAL2, carrying the 5' end of the gene. A third plasmid was constructed, carrying the entire PAL structural gene by amalgamating the inserts of the above two plasmids. This was achieved by isolating a 1.0kb FspI - PstI fragment from pPAL2, carrying the 5' end of PAL, and ligating it to a 1.25kb FspI - BamHI fragment carrying the 3' end of the gene isolated from pPAL1. The ligated DNA was then inserted into pUC9 cleaved with Pstl and BamHI (Fig. 4). The plasmid pPAL3 therefore carries the entire PAL structural gene, but lacks all 6 introns found in the natural R. toruloides chromosomal gene.

### 5. SYNTHESIS OF PAL PROTEIN

The fragment containing the complete intron-free PAL gene from pPAL3 has been cloned into the pUC plasmids in both orientations relative to the lac promoter, to give pPAL3 (pUC8) and pPAL4 (pUC9). In pPAL4, the PAL gene is in phase with the lacZ promoter from pUC9, and synthesis of PAL protein has been demonstrated in a plasmid-directed in vitro translation system. This is shown in Fig. 7. With the PAL gene in the opposite orientation (pPAL3) no PAL protein is produced. We are currently developing vector systems to enable us to express the PAL gene in Saccharomyces cerevisiae.

Ambrus, C.M., J.L. Ambrus, C. Hoarvath, H. Pedersen, S. Sharma, C. Kent, E. Mirand, R. Guthrie, and T. Paul. 1978. Phenylalanine depletion for the management of phenylketonuria: use of enzyme reactors with immobilized enzymes. Science 201: 837-839.

Attridge, T.H., C.B. Jonhson,and H. Smith. 1974. Density-labelling evidence for the phytochrome-mediated activation of phenylalanine ammonia-lyase in mustard cotyledons. Biochim. Biophys. Acta. 343: 440-451.

Boulivar, F., R.L. Rodriquez, P.J. Green, M.C. Betlach, H.L. Heyneker and H.W. Boyer. 1977. Construction and characterisation of new cloning vehicles II. A multipurpose cloning system. Gene 2: 95-113.

Clewell, D.B., and D.R. Helinski. 1969. Supercoiled circular DNA-protein complex in E. coli. Purification and induced conversion to an open circular form. Proc. Natl. Acad. Sci. USA 62: 1159.1166.

DeVries, J.K., and G. Zubay. 1967. DNA-directed peptide synthesis II. The synthesis of the -fragment of the enzyme β -galactosidase. Proc. Natl. Acad. Sci. USA 57: 1010-1012.

Gilbert, H.J., J.R. Stephenson, and M. Tully. 1983. Control of synthesis of functional mRNA coding for phenylalanine ammonia-lyase for Rhodosporidium toruloides. J. Bacteriol. 153: 1147-1154.

Gilbert, H.J., and M. Tully. 1982. Synthesis and degradation of phenylalanine ammonia-lyase of Rhodosporidium toruloides, J. Bacteriol. 150: 498-505.

Gilbert, H.J., I.N. Clarke, R.K. Gibson, J.R. Stephenson, and M. Tully. 1985. Molecular cloning of the phenylalanine ammonia lyase gene from Rhodosporidium toruloides in Escherichia coli K-12. J. Bacteriol. 161: 314-320.

Grunstein, M., and D.S. Hogness. 1975. Colony hybridisation: a method for the isolation of cloned DNA's that contain a specific gene. Proc. Natl. Acad. Sci. USA 72: 3961-3965.

Gubler, U., and B.J. Hoffman. 1983. A simple and very efficient method of generating cDNA libraries. Gene 25: 263-269.

Heidecker, G., and J. Messing. 1983. Sequence analysis of zein cDNA; obtained by an efficient mRNA cloning method. nacl. Acids. Res. 11: 4891-4906.

Holmes, D.S., and M. Quigley. 1981. A rapid boiling method for the preparation of bacterial plasmids. Anal.Biochem.114:193-197.

Laemmli, U.K. 1970. Cleavage of structural proteins during the assembly of the lead of bacteriophage T4. Nature 227: 680-685.

Marusich, W.C., R.A. jensen, and L.O. Zamir. 1981. Induction of L-phenylalanine ammonia-lyase during utilization of phenylalanine as carbon or nitrogen source in Rhodotorula glutinis. J. Bacteriol. 146: 1013-1019.

Messing, J., and J. Vieria. 1982. A new pair of M13 vectors for selecting either DNA strand of double-digest restriction fragments. 19: 269-276.

Minton, N.P., T. Atkinson, C.J. Bruton and R.F. Sherwood. 1984. The complete nucleotide sequence of the Pseudomonas gene coding for carboxypeptidase G₂. Gene 31: 31-38.

Mount, S.M. 1982. A catalogue of splice junction sequenceS. Nacl. Acids. Res. 10: 459-472.

Orbach, M.J., E.B. Porro, and C. Yanotsky. 1986. Cloning and characterisation of the gene for -tublin from a benomyl-resistant mutant of Neurospora crassa and its use as a dominant selectable marker. Mol. Cell. Biol. 6: 2452-2461.

Sanger, F., A.R. Coulson, B.G. Barrell, A.J.H. Smith and B.A. Roe. 1980. Cloning in single-stranded bacteriophage as an aid to rapid DNA sequencing. J. Mol. Biol. 143: 161-178.

Schroder, J., F. Kreuzaler, E. Schafer, and K. Hahlbrock. 1979. Concomitant induction of phenylalanine ammonia-lyase and flavanone synthase in irradiated plant cells. J. Biol. Chem. 254: 57-65.

Tan, S.C. 1980. Phenylalanine ammonia-lyase and the phenylalanine ammonia-lyase inactivating system: effects of light, temperature, and mineral deficiencies. Aust. J. Plant. Physiol. 1: 159-167.

Tully, M., and H.J. Gilbert. 1985. Transformation of Rhodosporidium toruloides. Gene 36: 235-240.

Vieria, J., and J. Messing. 1982. The pUC plasmids, an M13mp7-derived system for insertion mutagenesis and sequencing with synthetic universal primers. Gene 19: 259-260.

Yamada, S., K. Kabe, N. Izuo, K. Nakamichi, and I. Chibata. 1981. Production of L-phenylalanine from trans-cinnamic acid with Rhosdotorula qlutinis containing L-phenylalanine ammonia-lyase activity. Appl. Environ. Microbiol. 42: 773-778.

## Claims

1. An intron free structural gene, derived from a corresponding intron-containing structural gene from the eukaryotic microorganism R. toruloides, both genes encoding the same product, characterised in that the intron free gene is capable of expressing phenylalanine ammonia lyase ('PAL') or a polypeptide which displays PAL activity within a prokaryotic or eukaryotic microorganism.

2. An intron free gene characterised in that it has a structure identical to that of the PAL gene present in cDNA obtainable by either
(a) annealing total mRNA from PAL induced R. toruloides cells to oligo dT tailed pUC9, synthesising the first strand cDNA copy using reverse transcriptase in the presence of all 4 dNTPs, tailing the newly synthesised strands with oligo dC using terminal deoxynucleotidyl transferase, fractionating by an alkaline sucrose gradient, annealing single stranded plasmid DNA carrying cDNA sequences to denatured oligo dG tailed pUC9 and synthesising the second strand with Klenow DNA polymerase in the presence of all four dNTPs; or
(b) synthesising a cDNA strand from total mRNA using reverse transcriptase and a 19-mer primer of sequence GATGAGAGGGTTGTCGGTC complementary to PAL mRNA, nicking the RNA in the RNA-DNA hybrid so produced with RNAaseH and replacing it by DNA using E.coli DNA polymerase using the nicked RNA as a primer,
or a gene having a nucleotide sequence which codes for the same amino acids as this sequence.

3. A gene as claimed in claim 2 characterised in that it lacks some bases or includes some additional bases or has some of the codons replaced by other codons provided that the gene encodes PAL or a polypeptide displaying PAL activity.

4. A recombinant DNA molecule characterised in that it contains a gene as claimed in claim 1.

5. A recombinant DNA molecule characterised in that it contains a gene as claimed in claim 2 or claim 3.

6. A molecule as claimed in claim 4 or claim 5 characterised in that it is a plasmid.

7. A plasmid as claimed in claim 6 characterised in that it is a vector and also contains an expression control sequence operatively linked to the gene, and/or transcription/translation signals appropriate of PAL, or a polypeptide which displays PAL activity, from E. coli K12, Bacillus subtilis, Saccharomyces cerevisiae, Pseudomonas putida, Erwinia chrysanthemi or mammalian cell lines.

8. A plasmid as claimed in claim 7 characterised in that it contains a ribosome binding site upstream of the translational start of the gene and transcriptional signals derived from the S. cerevisiae phosphoglycerate kinase and mating factor genes placed 5' to the ribosome binding site.

9. A recombinant DNA molecule characterised in that it consists of a gene as claimed in claim 2 inserted into the plasmid pUC9 with the gene in phase with the lac Z promoter of pUC 9.

10. A host microorganism characterised in that it is E.coli transformed with a recombinant DNA molecule as claimed in claim 5.

11. A process for the preparation of a gene from which introns have been deleted characterised in that it includes the steps of:
(i) isolating PAL mRNA from a strain of R. toruloides.
(ii) synthesising two intron-free cDNA sequences from the mRNA, the two cDNA sequences each containing a portion of a gene which encodes PAL or a polypeptide which displays PAL activity, the two portions together containing the 3' and the 5' ends of the gene,
(iii) joining the two cDNA sequences to form an intron-free structural gene which encodes PAL or a polypeptide which displays PAL activity.

## Patentansprüche

1. Intron-freies Strukturgen, abgeleitet von einem entsprechenden Intron-haltigen Strukturgen aus dem eukaryontischen Mikroorganismus R. toruloides, wobei beide Gene für dasselbe Produkt kodieren, dadurch gekennzeichnet, daß das Intron-freie Gen Phenylalaninammoniaklyase ("PAL") oder ein PAL-Aktivität zeigendes Polypeptid in einem prokaryontischen oder eukaryontischen Mikroorganismus zu exprimieren vermag.

2. Intron-freies Gen, dadurch gekennzeichnet, daß es eine Struktur aufweist, die identisch ist mit der Struktur des PAL-Gens, das in cDNA enthalten ist,
erhältlich durch
(a) Reassoziieren der gesamten mRNA von PAL-induzierten Zellen von R. toruloides mit pUC9 mit Oligo(dT)-Schwänzen, Synthetisieren des ersten Strangs der cDNA-Kopie unter Verwendung von reverser Transkriptase in Gegenwart aller vier dNTPs, Anfügen von Oligo(dC)-Schwänzen an die neu synthetisierten Stränge unter Verwendung von terminaler Deoxynucleotidyl-Transferase, Fraktionieren mit einem alkalischen Saccharose-gradienten, Reassoziieren der einzelsträngigen Plasmid-DNA, die cDNA-Sequenzen trägt, mit denaturiertem pUC9 mit Oligo(dG)-Schwänzen, und Synthetisieren des zweiten Strangs mit Klenow-DNA-Polymerase in Gegenwart aller vier dNTPs, oder
(b) Synthetisieren eines cDNA-Strangs von der gesamten mRNA unter Verwendung von reverser Transkriptase und eines 19-mer-Primers mit der Sequenz GATGAGAGGGTTGTCGGTC, der zur PAL-mRNA komplementär ist, Erzeugen eines Einzelstrangabbruchs der RNA in dem so erzeugten RNA-DNA-Hybrid mit RNAaseH und Ersetzen der RNA durch DNA unter Verwendung von DNA-Polymerase von E. coli unter Verwendung der einzelstrangabgebrochenen RNA als Primer,
oder ein Gen, das eine Nucleotidsequenz aufweist, die die gleichen Aminosäuren wie diese Sequenz codiert.

3. Gen nach Anspruch 2, dadurch gekennzeichnet, daß ihm einige Basen fehlen, daß es einige zusätzliche Basen aufweist oder daß einige Codons durch andere Codons ersetzt sind unter der Voraussetzung, daß das Gen PAL oder ein PAL-Aktivität zeigendes Polypeptid codiert.

4. Rekombinantes DNA-Molekül, dadurch gekennzeichnet, daß es ein Gen nach Anspruch 1 enthält.

5. Rekombinantes DNA-Molekül, dadurch gekennzeichnet, daß es ein Gen nach Anspruch 2 oder 3 enthält.

6. Molekül nach Anspruch 4 oder 5, dadurch gekennzeichnet, daß es sich um ein Plasmid handelt.

7. Plasmid nach Anspruch 6, dadurch gekennzeichnet, daß es ein Vektor ist und ferner eine operativ mit dem Gen verknüpfte Expressionskontrollsequenz und/oder für PAL oder ein PAL-Aktivität zeigendes Polypeptid geeignete Transkriptions/Translations-Signale von E. coli K12, Bacillus subtilis, Saccharomyces cerevisiae, Pseudomonas putida, Erwinia chrysanthemi oder Säugetierzellinien enthält.

8. Plasmid nach Anspruch 7, dadurch gekennzeichnet, daß es eine Ribosomenbindungsstelle stromauf des Translationsstarts des Gens und von S. cerevisiae-Phosphoglyceratkinase stammende Transkriptionssignale sowie 5' zur Ribosomenbindungsstelle angeordnete Matingfaktorgene enthält.

9. Rekombinantes DNA-Molekül, dadurch gekennzeichnet, daß es aus einem in das Plasmid pUC9 eingefügten Gen nach Anspruch 2 besteht, wobei sich das Gen in Phase mit dem lac Z Promotor von pUC9 befindet.

10. Wirtsmikroorganismus, dadurch gekennzeichnet, daß es sich dabei um mit einem rekombinanten DNA-Molekül nach Anspruch 5 transformiertes E. coli handelt.

11. Verfahren zur Herstellung eines Gens, aus dem Introns deletiert worden sind, dadurch gekennzeichnet, daß es die folgenden Schritte umfaßt:
(i) Isolieren von PAL mRNA aus einem Stamm von R. toruloides,
(ii) Synthetisieren zweier Intron-freier cDNA-Sequenzen aus der mRNA, wobei die beiden cDNA-Sequenzen jeweils einen Teil eines für PAL oder ein PAL-Aktivität zeigendes Polypeptid kodierenden Gens und die beiden Teile zusammen das 3' und 5' Ende des Gens enthalten,
(iii) Verbinden der beiden cDNA-Sequenzen zur Bildung eines Intron-freien Strukturgens, das für PAL oder ein PAL-Aktivität zeigendes Polypeptid kodiert.

## Revendications

1. Gène de structure dépourvue d'intron, provenant d'un gène de structure correspondant, contenant de l'intron, du microorganisme eucaryote R. toruloides, les deux gênes codant pour le même produit, gène caractérisé en ce que le gène dépourvu d'intron est capable d'exprimer de la phénylalanine ammoniac lyase ("PAL") ou un polypeptide qui présente de l'activité de PAL dans un microorganisme procaryote ou eucaryote.

2. Gène dépourvu d'intron, caractérisé en ce qu'il a une structure identique à celle du gène PAL présent dans de l'ADNc que l'on peut obtenir par (a) annelage de l'ARNm total provenant de cellules de R. Toruloides induites par PAL donnant des oligo pUC9 à extrémités DT, synthèse de la copie d'ADNc de premiers brins en utilisant de la transcryptase inverse en présence de l'ensemble des quatre NTPd, extension homopolymérique des brins nouvellement synthétisés à l'aide d'oligo dC en utilisant de la désoxynucléotidyltransférase terminale, fractionnement par un gradient de saccharose alcalin, annelage de l'ADN, plasmide à brin simple portant des séquences d'ADNc pour dénaturer l'oligo pUC9 à extension homopolymérique dG, et synthèse du second brin avec de l'ADN polymérase de Klenov en présence de l'ensemble des quatre NTPd ; ou (b) synthèse d'un brin d'ADNc provenant de l'ARNn total, en utilisant de la transcryptase inverse et un amorceur mère 19 de séquence GATGAGAGGGTTGTCGGTC complémentaire de l'ARNm de PAL, coupure simple de brin de l'ARN dans l'hybride ARN-ADN ainsi produit avec de l'ARN-aseH et son remplacement par de l'ADN en utilisant de l'ADN polymérase de E.coli, en utilisant l'ARN coupé simple brin comme amorceur, ou un gène ayant une séquence de nucléotides qui code pour les mêmes amino-acides que cette séquence.

3. Gène tel que revendiqué à la revendication 2, caractérisé en ce qu'il ne comporte pas certaines bases ou en ce qu'il comporte quelques bases supplémentaires ou a certains des codons énumérés remplacés par d'autres codons, à la condition que le gène code pour PAL ou pour un polypeptide présentant de l'activité de PAL.

4. Molécule d'ADN recombinant, caractérisée en ce qu'elle contient un gène tel que revendiqué à la revendication 1.

5. Molécule d'ADN recombinant, caractérisée en ce qu'elle contient un gène tel que revendiqué à la revendication 2 ou à la revendication 3.

6. Molécule telle que revendiquée à la revendication 4 ou à la revendication 5, caractérisée en ce qu'elle est un plasmide.

7. Plasmide tel que revendiqué à la revendication 6, caractgérisé en ce qu'il est un vecteur et en ce qu'il contient également une séquence de maîtrise d'expression à liaison fonctionnelle avec le gène, et/ou des signaux de transcription/translation appropriées de PAL, ou d'un polypeptide présentant de l'activité de PAL, provenant de E. coli K12, Bacillus subtilis, Saccharomyces cerevisiae, Pseudomonas putida, Erwinia chrysanthemi ou des lignées de cellules de mammifères.

8. Plasmide tel que revendiqué à la revendication 7, caractérisé en ce qu'il contient un site de fixation de robosome en amont du départ de translation du gène et des signaux de transcription provenant de S. cerevisiae phosphoglycérate kinase et des gènes d'un facteur de correspondance placé en 5' par rapport au site de liaison de fixation de robosome.

9. Molécule d'ADN recombinant, caractérisée en ce qu'elle consiste en un gène tel que revendiqué à la revendication 2, inséré dans le plasmide pUC 9, ce gène étant en phase avec le promoteur lac z de pUC 9.

10. Microorganisme hôte, caractérisé en ce qu'il est E. coli transformé avec une molécule d'ADN recombinant telle que revendiquée à la revendication 5.

11. Procédé pour la préparation d'un gène dont des introns ont été enlevés, procédé caractérisé en ce qu'il comprend les étapes consistant :
(i) à isoler de l'ARNm de PAL à partir d'une souche de R. toruloides ;
(ii) à effectuer la synthèse de deux séquences d'ADNc dépourvues d'intron, à partir d'ARNm, les deux séquences d'ADNc contenant chacune une portion d'un gène qui code pour PAL ou pour un polypeptide qui présente de l'activité de PAL, les deux portions contenant ensemble les extrémités 3' et 5' du gène ;
(iii) à relier les deux séquences d'ADNc pour former un gène de structure dépourvue d'intron, qui code pour PAL ou pour un polypeptide présentant de l'activité de PAL.
